# EUROPEAN PATENT SPECIFICATION

(11) **EP 0 369 823 B1**
(45) Date of publication and mention of the grant of the patent: **16.06.1993**
(21) Application number: 89311959.4
(22) Date of filing: 17.11.1989
(51) Int. Cl.: C07C 50/02, C07C 46/08

(54) **Method for the preparation of 2,3,5-trimethylbenzoquinone**
Verfahren zur Herstellung von 2,3,5-Trimethylbenzochinon
Procédé de préparation de triméthyl-2,3,5 benzoquinone

(30) Priority: 18.11.1988 JP 291668/88; 05.04.1989 JP 86030/89
(43) Date of publication of application: 23.05.1990
(73) Proprietor: THE STATE OF JAPAN, as Represented by the DIRECTOR GENERAL of the AGENCY of INDUSTRIAL SCIENCE and TECHNOLOGY, Tokyo 100 (JP)
(72) Inventor: Takehira, Katsuomi, Tsukuba-shi Ibaraki-ken (JP); Orita, Hideo, Tsukuba-shi Ibaraki-ken (JP); Shimizu, Masao, Tsukuba-shi Ibaraki-ken (JP); Hayakawa, Takashi, Tsukuba-shi Ibaraki-ken (JP)
(74) Representative: Baverstock, Michael George Douglas

(56) References cited:
- EP-A- 0 104 937
- EP-A- 0 105 067
- EP-A- 0 294 584
- DE-A- 2 444 234
- US-A- 4 257 968
- US-A- 4 545 937
- US-A- 4 828 762

## Description

The present invention relates to a method for the preparation of 2,3,5-trimethyl benzoquinone, which is referred to as TMQ hereinbelow, or, more particularly, to a method for the preparation of TMQ by the oxidation of 2,3,6-trimethyl phenol, which is referred to as TMP hereinbelow, with oxygen in the presence of a catalyst.

TMQ is an important organic compound as an intermediate in the synthesis of various kinds of organic compounds such as synthetic vitamin E and the like. A most conventional synthetic method for the preparation of TMQ is oxidation of sulfonated TMP by using an inorganic oxidizing agent such as manganese dioxide, potassium permanganate, lead oxide and the like according to the disclosure in West German Patents No. 1932362 and No. 2225543. This conventional two-step method including sulfonation and oxidation is industrially not advantageous due to the high costs along with production of a large amount of by-products as noxious waste materials to be disposed of without problems of environmental pollution as well as the toxicity of the catalytic heavy metal compounds. Accordingly, this method is desired to be replaced with an improved method of low costs free from the above mentioned problems.

Many attempts and proposals have been made of a one-step method for the preparation of TMQ by the oxidation of TMP using different oxidizing agents including nitric acid disclosed in Japanese Patent Publication 56-95145, perbenzoic acid disclosed in Japanese Patent Publication 59-39847, hypohalogenous acids disclosed in Japanese Patent Publication 60-81135, hydrogen peroxide disclosed in European Patent Application 107176. These methods are still not free from various problems such as evolution of toxic gases, expensiveness of the oxidizing agent, formation of noxious by-products to be disposed of and the like.

Alternatively, a method for the preparation of TMQ is now under way of development using oxygen as the oxidizing agent of TMP in the presence of a catalyst. The efficiency of this method naturally depends on the activity of the catalyst or catalyst system used so that various proposals have been made for the catalyst. For example, Japanese Patent Publication 56-26647 proposes a cobalt complex as the catalyst. This method, however, is not practically applicable due to the extremely short life of the catalyst although the catalytic activity at the initial stage is sufficiently high. Use of a copper halide as the catalyst is also proposed to give high reactivity and selectivity of the catalytic oxidation reaction but the method has several defects to be resolved when the method is industrially practiced. For example, the method proposed in Japanese Patent Kokai 49-3664, in which the oxidation reaction using a copper salt as the catalyst is performed in a nitrile compound or tertiary amide compound as a solvent, is not an industrially efficient method because the yield of the product TMQ is about 75% at the highest and the product TMQ must be isolated from the reaction mixture in a quite troublesome process since the reaction mixture usually contains a substantial amount of polymeric by-products such as poly(phenylene oxide) and the like. Japanese Patent Publication 53-17585 proposes a method in which TMP dissolved in an organic solvent is oxidized with oxygen in the presence of a catalyst system composed of copper ions and halogen ions. Despite the high yield of the desired product, this method is also not industrially applicable because of the low productivity as a consequence of the very low catalytic activity which necessitates the use of a large amount of the catalyst sometimes in an equimolar amount to the starting TMP and the long time taken for completion of the oxidation reaction. Moreover, the most serious problem prohibiting the industrial application of this method is that a troublesome and high-cost process must be undertaken for the recycling and reuse of the catalyst used in a large amount with a large consumption of plant utility supplies. This problem has been the subject matter of inventions disclosed, for example, in Japanese Patent Kokai 50-93931 and 59-225137 which, however, are directed merely to the improvement in the process of recycling and reuse of the catalyst from the preceding run. Namely, no improvement can be seen therein relative to the activity of the catalyst which determines the reaction velocity.

The present invention accordingly has an object to provide a novel and improved method for the preparation of TMQ by the oxidation of TMP with oxygen in the presence of a catalyst without the problems and disadvantages in the above described prior art methods.

Thus, the method of the present invention for the preparation of TMQ by the oxidation of TMP comprises: oxidizing TMP dissolved in an alcoholic organic solvent by contacting with molecular oxygen in the presence of a catalyst system which is a combination of a copper compound and a nitrogen-containing compound selected from
(a) salts of a hydroxylamine compound with an inorganic acid, (b-1) oxime compounds, (b-2) salts of an oxime compound with an inorganic acid and (c) salts of an amine compound with an inorganic acid.

It is noted that selection of the organic solvent as the reaction medium of the above described oxidation reaction has some significance on the efficiency of the catalytic reaction. Preferred solvents or solvent mixtures include lower aliphatic branched alcohols having 3 to 6 carbon atoms in a molecule, mixtures of an aromatic hydrocarbon solvent and a lower aliphatic alcohol having 1 to 6 carbon atoms and aliphatic alcohols having 4 to 10 carbon atoms.

As is described above, the method of the present invention is basically a catalytic oxidation reaction of TMP dissolved in an organic solvent with molecular oxygen. The reaction can be performed easily by merely agitating a solution of TMP in an organic solvent containing the catalyst system under an atmosphere of oxygen as the oxidizing agent at a temperature in the range from room temperature to 200 °C so that the method is industrially very advantageous in respect of productivity along with the absence of problems relative to safety and environmental pollution.

The oxidizing agent in the inventive method is molecular oxygen which can be pure oxygen, atmospheric air as such or oxygen-enriched air. The oxidation reaction of the inventive method can proceed even under atmospheric pressure but it is optional to conduct the reaction under a super-atmospheric pressure, for example, up to 30 kg/cm² with an object to promote the oxidation reaction.

The catalyst or catalyst system used in the inventive method is a combination of a copper compound and a nitrogen-containing compound specified above. The type of the copper compound as one of the catalytic ingredients is not particularly limitative including inorganic copper salts and organic complexes of copper. Particularly preferred copper compounds include halogen or chlorine compounds of copper such as copper (I) chloride, copper (II) chloride and the like in respect of the high catalytic activity.

The other catalytic ingredient forming the catalyst system as combined with the above mentioned copper compound is a nitrogen-containing compound selected from the classes including (a) salts of a hydroxylamine compound with an inorganic acid, (b-1) oxime compounds, (b-2) salts of an oxime compound with an inorganic acid and (c) salts of an amine compound with an inorganic acid. It is optional to use these compounds, belonging to the same class or to different classes, as a combination of two kinds or more according to need. It is noted that a mixture of a nitrogen-containing compound, e.g., hydroxylamine compound, oxime compound and amine compound, and an inorganic acid is equivalent to the salt of the nitrogen-containing compound.

The above mentioned hydroxylamine compound includes: hydroxylamine; N,N-dialkyl hydroxylamine, e.g., N,N-dimethyl hydroxylamine, N,N-diethyl hydroxylamine and the like; N-alkyl hydroxcylamines, e.g., N-methyl hydroxylamine; O-alkyl hydroxylamines, e.g., O-methyl hydroxylamine and the like; N-hydroxy imides, e.g., N-hydroxy succiimide, N-hydroxy phthalimide and the like; and derivatives of hydroxylamine, e.g., hydroxy urea, hydroxy urethane and the like, of which hydroxylamine, hydroxy urea and N,N-dialkyl hydroxylamines, of which the alkyl group is a lower alkyl group having 1 to 4 carbon atoms, are particularly preferred. The inorganic acid to form a salt or to be used in combination with the hydroxylamine compound is not particularly limitative including various kinds of halogen-containing acids, sulfuric acid and the like, of which hydrochloric acid and sulfuric acid are preferred in respect of the relatively high catalytic activity. When the inorganic acid is used in combination with the hydroxylamine compound, the ratio of the amounts of the acid and hydroxylamine compound is not particularly limitative but it is usually in the range from 0.2 to 5 moles of the inorganic acid per mole of the hydroxylamine compound to give a good catalytic activity. The ratio of the amounts of the hydroxylamine compound and the copper compound is also not particularly limitative but it is usually in the range from 0.3 to 3 moles of the hydroxylamine compound per mole of the copper compound to give a relatively high reaction velocity. Too large or too small amounts of the hydroxylamine compound relative to the copper compound may cause a decrease in the reaction velocity.

The above mentioned oxime compound to be used as such or in the form of an inorganic acid salt in combination with the copper compound includes ketoximes derived from dialkyl ketones, e.g., acetone, methyl ethyl ketone, diethyl ketone and the like, cyclic ketones, e.g., cyclohexanone, cyclooctanone and the like; aromatic ketones, e.g., acetophenone, propiophenone and the like, diketones, e.g., acetyl acetone and the like, and cyclic diketones, e.g., dimedone and the like, as well as aldoximes derived from aliphatic aldehydes, e.g., formaldehyde, acetaldehyde, propionaldehyde and the like, and aromatic aldehydes, e.g., benzaldehyde, phenyl acetaldehyde and the like, of which particularly preferred are those having a relatively small molecular weight such as acetoaldoxime, benzaldoxime, acetone oxime, 2-butanone oxime and the like.

It is not always necessary that the above described oxime compound is used in the form of an inorganic acid salt or in combination with an inorganic acid but the oxime compound is effective as such although better results can be obtained by using the oxime compound in the form of an inorganic acid salt or in combination with an inorganic acid than by using the oxime compound as such in respect of the catalytic activity. The inorganic acid to form a salt or to be used in combination with the oxime compound is not particularly limitative including various kinds of halogen-containing acids, sulfuric acid and the like, of which hydrochloric acid and sulfuric acid are preferred in respect of the relatively high catalytic activity. When the inorganic acid is used in combination with the oxime compound, the ratio of the amounts of the acid and oxime compound is not particularly limitative but it is usually in the range from 0.2 to 5 moles of the inorganic acid per mole of the oxime compound to give a good catalytic activity. The ratio of the amounts of the oxime compound and the copper compound is also not particularly limitative but it is usually in the range from 0.3 to 3 moles of the oxime compound per mole of the copper compound to give a relatively high reaction velocity. A too large or too small amount of the oxime compound relative to the copper compound may cause a decrease in the reaction velocity.

The amine compound to be combined with the copper compound in the form of an inorganic acid salt or in combination with an inorganic acid includes primary, secondary and tertiary amines as well as cyclic amines. Derivatives of an amine compound such as amino alcohols, amino acids and the like are also suitable without particular limitations. As a general rule, amine compounds having a relatively small molecular weight are preferred. The inorganic acid to form a salt or to be used in combination with the amine compound is not particularly limitative including various kinds of halogen-containing acids, sulfuric acid and the like, of which hydrochloric acid and sulfuric acid are preferred in respect of the relatively high catalytic acitivity. When the inorganic acid is used in combination with the amine compound, the ratio of the amounts of the acid and amine compound is not particularly limitative but it is usually in the range from 0.2 to 5 moles of the inorganic acid per mole of the amine compound to give a good catalytic activity. The ratio of the amounts of the amine compound and the copper compound is also not particularly limitative but it is usually in the range from 0.3 to 3 moles of the amine compound per mole of the copper compound to give a relatively high reaction velocity. Too large or too small amounts of the amine compound relative to the copper compound may cause a decrease in the reaction velocity.

Though not particularly limitative, the amount of the above described catalyst system in the reaction mixture in the inventive method is usually in the range from 0.01 to 0.5 mole or, preferably, from 0.01 to 0.1 mole of the copper compound per mole of the TMP as the starting material. When the amount of the catalyst system is too small, insufficient reaction velocity can be obtained. Use of an excessively large amount of the catalyst is undesirable in view of the troubles encountered in the separation of the catalyst from the reaction mixture after completion of the reaction.

The oxidation reaction of the inventive method is performed by dissolving or dispersing the starting TMP and the catalytic ingredients in an organic solvent. The concentration of TMP in the reaction mixture is preferably in the range from 0.05 to 5 M or, more preferably, from 0.1 to 1.0 M. Organic solvents suitable for use in the present invention include alcohols and monoalkyl ethers of glycols.

In particular, alcohols having 3 to 10 carbon atoms in a molecule are preferable. These organic solvents can be used either singly or as a mixture of two kinds or more according to need.

Further particularly, quite satisfactory results could be obtained by using an alcohol of a branched molecular structure having 3 to 6 carbon atoms in a molecule. Such an alcohol is exemplified by isopropyl alcohol, isobutyl alcohol, sec-butyl alcohol, tert-butyl alcohol, isoamyl alcohol, sec-amyl alcohol, tert-amyl alcohol, pentan-3-ol, hexan-2-ol, 3-methylpentan-3-ol and the like, of which tertiary alcohols such as tert-butyl alcohol, tert-amyl alcohol and the like are preferred.

Another class of organic solvents capable of giving results as satisfactory as those obtained by use of the above mentioned branched alcohols include combinations of an aromatic hydrocarbon solvent and an aliphatic lower alcohol having 1 to 6 carbon atoms in a molecule. The aromatic hydrocarbon solvent should preferably have a relatively low boiling point and be resistant against oxidation as exemplified by benzene, toluene, xylene and the like though not particularly limitative. The aliphatic lower alcohol having 1 to 6 carbon atoms in a molecule is exemplified by methyl alcohol, ethyl alcohol, n-propyl alcohol, isopropyl alcohol, n-butyl alcohol, sec-butyl alcohol, tert-butyl alcohol, n-amyl alcohol, sec-amyl alcohol, pentan-3-ol, tert-amyl alcohol and the like. The mixing ratio of the aromatic hydrocarbon solvent to the aliphatic lower alcohol is usually in the range from 0.2 to 1.5 by volume or, preferably, from 0.25 to 0.8 by volume although the exact mixing ratio should be adequately selected depending on the particular combination of the solvents.

The above described preferable solvents are quite effective in dissolving the catalyst system and the TMP as the starting material as well as the molecular oxygen as the oxidizing agent so that the oxidation reaction can proceed very smoothly by merely contacting the TMP with the oxidizing agent dissolved in the solution containing the catalyst system.

The catalytic ingredients forming the catalyst system can be used by being directly dissolved in the organic solvent. When the organic solvent is an aliphatic alcohol having 4 to 10 carbon atoms in a molecule and little soluble in water, such as butyl, pentyl, hexyl and octyl alcohols including isomeric mixtures thereof, it is optional that the catalytic ingredients are introduced into the reaction mixture in the form of an aqueous solution prepared separately so that the oxidation reaction proceeds in a heterogeneous reaction mixture under agitation. The concentration of the catalytic ingredients in the aqueous solution is in the range from 0.05 to 5 M or, preferably, from 0.1 to 1.0 M relative to the copper compound.

The oxidation reaction of the inventive method is performed by keeping the reaction mixture at a temperature in the range from room temperature to about 200 °C or, preferably, from room temperature to 80 °C. The reaction velocity would naturally be small at lower temperatures. An excessively high temperature is undesirable due to the eventual vaporization loss of the solvent and an increase in the amount of the by-products produced by side reactions. At any rate, it is important that the reactor is equipped with a powerful agitating means and an aerating means in order to ensure good contacting of the starting material, molecular oxygen and catalytic ingredients dissolved in the solvent. The oxidation reaction is complete usually within 1 to 10 hours although the time taken for completion of the oxidation reaction naturally depends on various factors such as the reaction temperature, pressure of oxygen, types and amounts of the catalytic ingredients and so on.

To summarize the above given description of the inventive method, the present invention provides a very advantageous industrial method for the preparation of TMQ from TMP since the reaction can be performed at a very high reaction velocity to give the product in a high yield by using molecular oxygen as the oxidizing agent with inexpensive catalytic ingredients dissolved in an also inexpensive organic solvent under moderate reaction conditions. Moreover, the inventive method is free from the very troublesome problem in the prior art methods in which a large amount of the catalyst must be recovered from the reaction mixture and recycled. When the catalyst ingredients are introduced to the reaction mixture in the form of an aqueous solution, the reaction mixture forms an emulsified phase of the aqueous catalyst solution in the organic solvent under vigorous agitation so that the oxidation reaction proceeds on the surface of the dispersed droplets of the aqueous catalyst solution. The emulsification of the aqueous catalyst solution is facilitated by virtue of the combination of the oleophilic aromatic hydrocarbon solvent and the hydrophilic aliphatic lower alcohol or by the surface-active nature of the aliphatic alcohol having an oleophilic alkyl group and a hydrophilic hydroxy group. When agitation of the reaction mixture is interrupted after completion of the reaction, the reaction mixture is rapidly separated into an organic solution and an aqueous solution from which the catalyst ingredients can be recovered and reused. The organic solution is subjected to distillation so as to recover the organic solvent and isolate the TMQ as the desired product.

In the following, the method of the invention is described in more detail by way of examples, in which the conversion of the starting TMP and the yield of the product TMQ were determined by the method of gas chromatography using 1,2-dichlorobenzene as an internal standard.

### Example 1.

Into a glass vessel of 10 ml capacity were introduced 2 m moles of TMP, 0.1 m mole of copper (II) chloride dihydrate, 0.1 m mole of hydroxylamine hydrochloride and 2 ml of n-hexyl alcohol to form a reaction mixture which was kept at 60 °C under an oxygen pressure of 860 mm Hg to effect the oxidation reaction. The volume of oxygen absorbed by the reaction mixture was determined periodically by using a gas burette to find that about 2 m moles of oxygen had been absorbed after 1.0 hour from the start of the reaction. Thereafter, the oxygen gas was no longer absorbed by the reaction mixture. After keeping the reaction mixture for additional 1 hour under the same conditions as above to complete the reaction, the reaction mixture was analyzed for the conversion of the starting TMP and the yield of the product TMQ to find that the conversion of the TMP was 100% and the yield of the TMQ was 83.2% corresponding to a selectivity of 83.2%.

### Example 2.

The reaction conditions were substantially the same as in Example 1 except that the reaction temperature was 40 °C instead of 60 °C. Absorption of the oxygen gas came near to termination after 1.5 hours of the reaction when about 2 m moles of oxygen had been absorbed. After keeping for additional 1.5 hours to complete the reaction, the reaction mixture was analyzed to find that the conversion of the TMP was 100% and the yield of the TMQ was 82.3% corresponding to a selectivity of 82.3%.

### Example 3.

The reaction conditions were substantially the same as in Example 1 except that the amounts of TMP, copper (II) chloride dihydrate and hydroxylamine hydrochloride were 3 m moles, 0.05 m mole and 0.2 m mole, respectively. Absorption of the oxygen gas came near to termination after 1 hour of the reaction when about 3 m moles of oxygen had been absorbed. After keeping for additional 1 hour to complete the reaction, the reaction mixture was analyzed to find that the conversion of the TMP was 100% and the yield of the TMQ was 82.1% corresponding to a selectivity of 82.1%.

### Example 4.

The reaction conditions were substantially the same as in Example 1 except that the amounts of copper (II) chloride dihydrate and hydroxylamine hydrochloride were each 0.2 m mole. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 85.4% corresponding to a selectivity of 85.4%.

### Example 5.

The reaction conditions were substantially the same as in Example 4 except that the hydroxylamine hydrochloride was replaced with the same molar amount of hydroxylamine sulfate. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 86.8% corresponding to a selectivity of 86.8%.

### Example 6.

The reaction conditions were substantially the same as in Example 4 except that the n-hexyl alcohol was replaced with the same volume of n-octyl alcohol. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 79.1% corresponding to a selectivity of 79.1%.

### Example 7.

The reaction conditions were substantially the same as in Example 1 except that the amount of copper (II) chloride dihydrate was 0.2 m mole, the reaction temperature was 40 °C and the hydroxylamine hydrochloride was replaced with the same molar amount of hydroxylamine sulfate. Absorption of the oxygen gas came near to termination after 1.5 hours of the reaction when about 2 m moles of oxygen had been absorbed. After keeping for additional 1.5 hours to complete the reaction, the reaction mixture was analyzed to find that the conversion of the TMP was 100% and the yield of the TMQ was 84.3% corresponding to a selectivity of 84.3%.

### Example 8.

The reaction conditions were substantially the same as in Example 3 except that the amount of copper (II) chloride dihydrate was 0.1 m mole and the hydroxylamine hydrochloride was replaced with the same molar amount of hydroxylamine sulfate. Absorption of the oxygen gas came near to termination after 1 hour of the reaction when about 3 m moles of oxygen had been absorbed. After keeping for additional 3 hours to complete the reaction, the reaction mixture was analyzed to find that the conversion of the TMP was 100% and the yield of the TMQ was 82.5% corresponding to a selectivity of 82.5%.

### Example 9.

The reaction conditions were substantially the same as in Example 2 except that the amount of copper (II) chloride dihydrate was 0.2 m mole and the hydroxylamine hydrochloride was replaced with the same molar amount of hydroxylamine sulfate. Absorption of the oxygen gas came near to termination after 1.5 hours of the reaction when about 2 m moles of oxygen had been absorbed. After keeping for additional 2 hours to complete the reaction, the reaction mixture was analyzed to find that the conversion of the TMP was 100% and the yield of the TMQ was 80.5% corresponding to a selectivity of 80.5%.

### Example 10.

The reaction conditions were substantially the same as in Example 3 except that the amounts of copper (II) chloride dihydrate and hydroxylamine hydrochloride were 0.1 m mole and 0.4 m mole, respectively. Absorption of the oxygen gas came near to termination after 1 hour of the reaction when about 3 m moles of oxygen had been absorbed. After keeping for additional 3 hours to complete the reaction, the reaction mixture was analyzed to find that the conversion of the TMP was 100% and the yield of the TMQ was 79.1% corresponding to a selectivity of 79.1%.

### Example 11.

The reaction conditions were substantially the same as in Example 4 except that 0.2 m mole of the hydroxylamine hydrochloride was replaced with a combination of 0.2 m mole of N,N-diethyl hydroxylamine and 0.017 ml of a 36% hydrochloric acid corresponding to 0.2 m mole of hydrogen chloride. Absorption of the oxygen gas came to termination after 1.5 hours of the reaction when about 2 m moles of oxygen had been absorbed. After keeping for additional 1.5 hours to complete the reaction, the reaction mixture was analyzed to find that the conversion of the TMP was 100% and the yield of the TMQ was 77.3% corresponding to a selectivity of 77.3%.

### Example 12.

The reaction conditions were substantially the same as in Example 4 except that the hydroxylamine hydrochloride was replaced with the same molar amount of N,N-dimethyl hydroxylamine hydrochloride. About 2 m moles of oxygen were absorbed during the reaction for 9 hours. The reaction mixture was analyzed to find that the conversion of the TMP was 100% and the yield of the TMQ was 70.2% corresponding to a selectivity of 70.2%.

### Example 13.

The reaction conditions were substantially the same as in Example 4 except that 0.2 m mole of the hydroxylamine hydrochloride was replaced with a combination of 0.2 m mole of hydroxy urea and 0.017 ml of a 36% hydrochloric acid corresponding to 0.2 m mole of hydrogen chloride. About 2 m moles of oxygen were absorbed during the reaction for 7 hours. The reaction mixture was analyzed to find that the conversion of the TMP was 99.0% and the yield of the TMQ was 82.1% corresponding to a selectivity of 82.1%.

### Comparative Example 1.

The reaction conditions were substantially the same as in Example 11 excepting omission of the 36% hydrochloric acid. About 2 m moles of oxygen were absorbed during the reaction for 4 hours. The reaction mixture was analyzed to find that the conversion of the TMP was 100% and the yield of the TMQ was 63.1% corresponding to a selectivity of 63.1%.

### Comparative Example 2.

The reaction conditions were substantially the same as in Example 1 except that the hydroxylamine hydrochloride was replaced with the same molar amount of lithium chloride. About 0.8 m mole of oxygen was absorbed during the reaction for 5 hours. The reaction mixture was analyzed to find that the conversion of the TMP was 50.4% and the yield of the TMQ was 20.3% corresponding to a selectivity of 40.3%.

### Comparative Example 3.

The reaction conditions were substantially the same as in Example 3 except that the hydroxylamine hydrochloride was omitted and the amount of the copper (II) chloride dihydrate was 0.1 m mole. About 0.3 m mole of oxygen was absorbed during the reaction for 5 hours. The reaction mixture was analyzed to find that the conversion of the TMP was 10.7% and the yield of the TMQ was 3.8% corresponding to a selectivity of 35.5%.

### Comparative Example 4.

The reaction conditions were substantially the same as in Comparative Example 2 except that the reaction mixture was further admixed with 0.009 ml of a 36% hydrochloric acid corresponding to 0.1 m mole of hydrogen chloride. About 0.8 m mole of oxygen was absorbed during the reaction for 5 hours. The reaction mixture was analyzed to find that the conversion of the TMP was 46.7% and the yield of the TMQ was 25.7% corresponding to a selectivity of 55.0%.

### Example 14.

The reaction conditions were substantially the same as in Example 4 except that 0.2 m mole of the hydroxylamine hydrochloride was replaced with a combination of 0.2 m mole of acetone oxime and 0.017 ml of a 36% hydrochloric acid corresponding to 0.2 m mole of hydrogen chloride. Absorption of the oxygen gas came near to termination after 1.0 hour of the reaction when about 2 m moles of oxygen had been absorbed. After keeping for additional 1.5 hours to complete the reaction, the reaction mixture was analyzed to find that the conversion of the TMP was 100% and the yield of the TMQ was 84.5% corresponding to a selectivity of 84.5%.

### Example 15.

The reaction conditions were substantially the same as in Example 14 except that 0.017 ml of the 36% hydrochloric acid was replaced with 0.011 ml of a 98% sulfuric acid corresponding to 0.2 m mole of the acid. Absorption of the oxygen gas came near to termination after 2.0 hours of the reaction when about 2 m moles of oxygen had been absorbed. After keeping for additional 2.0 hours to complete the reaction, the reaction mixture was analyzed to find that the conversion of the TMP was 100% and the yield of the TMQ was 80.3% corresponding to a selectivity of 80.3%.

### Example 16.

The reaction conditions were substantially the same as in Example 14 excepting omission of the hydrochloric acid. Absorption of the oxygen gas came near to termination after 1.5 hours of the reaction when about 2 m moles of oxygen had been absorbed. After keeping for additional 2.0 hours to complete the reaction, the reaction mixture was analyzed to find that the conversion of the TMP was 100% and the yield of the TMQ was 80.4% corresponding to a selectivity of 80.4%.

### Example 17.

The reaction conditions were substantially the same as in Example 14 except that the amount of the copper (II) chloride dihydrate was 0.1 m mole. Absorption of the oxygen gas came near to termination after 1.5 hours of the reaction when about 2 m moles of oxygen had been absorbed. After keeping for additional 2.0 hours to complete the reaction, the reaction mixture was analyzed to find that the conversion of the TMP was 100% and the yield of the TMQ was 77.4% corresponding to a selectivity of 77.4%.

### Example 18.

The reaction conditions were substantially the same as in Example 14 except that the amount of the copper (II) chloride dihydrate was 0.1 m mole and the volume of the 36% hydrochloric acid was decreased to 0.009 ml corresponding to 0.1 m mole of hydrogen chloride. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 78.4% corresponding to a selectivity of 78.4%.

### Example 19.

The reaction conditions were substantially the same as in Example 14 except that the amounts of the copper (II) chloride dihydrate and the acetone oxime were 0.1 m mole and 0.3 m mole, respectively, and the volume of the 36% hydrochloric acid was decreased to 0.009 ml corresponding to 0.1 m mole of hydrogen chloride. Absorption of the oxygen gas came near to termination after 1 hour of the reaction when about 2 m moles of oxygen had been absorbed. After keeping for additional 2 hours to complete the reaction, the reaction mixture was analyzed to find that the conversion of the TMP was 100% and the yield of the TMQ was 81.2% corresponding to a selectivity of 81.2%.

### Example 20.

The reaction conditions were substantially the same as in Example 14 except that the n-hexyl alcohol was replaced with the same volume of n-octyl alcohol. Absorption of the oxygen gas came near to termination after 1 hour of the reaction when about 2 m moles of oxygen had been absorbed. After keeping for additional 1.5 hours to complete the reaction, the reaction mixture was analyzed to find that the conversion of the TMP was 100% and the yield of the TMQ was 76.2% corresponding to a selectivity of 76.2%.

### Example 21.

The reaction conditions were substantially the same as in Example 14 except that the amounts of the starting TMP and copper (II) chloride dihydrate were 3 m moles and 0.1 m mole, respectively. Absorption of the oxygen gas came near to termination after 1.5 hours of the reaction when about 3 m moles of oxygen had been absorbed. After keeping for additional 2.5 hours to complete the reaction, the reaction mixture was analyzed to find that the conversion of the TMP was 100% and the yield of the TMQ was 79.3% corresponding to a selectivity of 79.3%.

### Example 22.

The reaction conditions were substantially the same as in Example 14 except that the amount of the starting TMP was 3 m moles and the volume of the 36% hydrochloric acid was decreased to 0.009 ml corresponding to 0.1 m mole of hydrogen chloride. Absorption of the oxygen gas came near to termination after 1.5 hours of the reaction when about 2 m moles of oxygen had been absorbed. After keeping for additional 2.5 hours to complete the reaction, the reaction mixture was analyzed to find that the conversion of the TMP was 100% and the yield of the TMQ was 81.5% corresponding to a selectivity of 81.5%.

### Example 23.

The reaction conditions were substantially the same as in Example 21 except that the amounts of the acetone oxime and the 36% hydrochloric acid were 0.1 m mole and 0.009 ml corresponding to 0.1 m mole of hydrogen chloride, respectively. Absorption of the oxygen gas came near to termination after 1.5 hours of the reaction when about 3 m moles of oxygen had been absorbed. After keeping for additional 2.5 hours to complete the reaction, the reaction mixture was analyzed to find that the conversion of the TMP was 100% and the yield of the TMQ was 80.9% corresponding to a selectivity of 80.9%.

### Example 24.

The reaction conditions were substantially the same as in Example 14 except that the reaction temperature was 40 °C instead of 60 °C. Absorption of the oxygen gas came near to termination after 2.5 hours of the reaction when about 2 m moles of oxygen had been absorbed. After keeping for additional 2.5 hours to complete the reaction, the reaction mixture was analyzed to find that the conversion of the TMP was 100% and the yield of the TMQ was 79.4% corresponding to a selectivity of 79.4%.

### Example 25.

The reaction conditions were substantially the same as in Example 14 except that the acetone oxime was replaced with the same molar amount of 2-butanone oxime. Absorption of the oxygen gas came near to termination after 1 hour of the reaction when about 2 m moles of oxygen had been absorbed. After keeping for additional 2 hours to complete the reaction, the reaction mixture was analyzed to find that the conversion of the TMP was 100% and the yield of the TMQ was 75.5% corresponding to a selectivity of 75.5%.

### Example 26.

The reaction conditions were substantially the same as in Example 25 excepting omission of the hydrochloric acid. Absorption of the oxygen gas came near to termination after 1 hour of the reaction when about 2 m moles of oxygen had been absorbed. After keeping for additional 2 hours to complete the reaction, the reaction mixture was analyzed to find that the conversion of the TMP was 100% and the yield of the TMQ was 74.3% corresponding to a selectivity of 74.3%.

### Example 27.

The reaction conditions were substantially the same as in Example 14 except that the acetone oxime was replaced with the same molar amount of benzaldoxime. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 83.2% corresponding to a selectivity of 83.2%.

### Example 28.

The reaction conditions were substantially the same as in Example 27 excepting omission of the hydrochloric acid. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 80.1% corresponding to a selectivity of 80.1%.

### Example 29.

The reaction conditions were substantially the same as in Example 14 except that the acetone oxime was replaced with the same molar amount of acetaldoxime. Absorption of the oxygen gas came near to termination after 1 hour of the reaction when about 2 m moles of oxygen had been absorbed. After keeping for additional 1 hour to complete the reaction, the reaction mixture was analyzed to find that the conversion of the TMP was 100% and the yield of the TMQ was 84.2% corresponding to a selectivity of 84.2%.

### Example 30.

The reaction conditions were substantially the same as in Example 29 excepting omission of the hydrochloric acid. About 2 m moles of oxygen were absorbed during the reaction for 5 hours. The reaction mixture was analyzed to find that the conversion of the TMP was 100% and the yield of the TMQ was 80.4% corresponding to a selectivity of 80.4%.

### Example 31.

The reaction conditions were substantially the same as in Example 16 except that the acetone oxime was replaced with the same molar amount of cyclohexanone oxime. About 2 m moles of oxygen were absorbed during the reaction for 6 hours. The reaction mixture was analyzed to find that the conversion of the TMP was 97.1% and the yield of the TMQ was 72.5% corresponding to a selectivity of 74.7%.

### Example 32.

The reaction conditions were substantially the same as in Example 4 except that the hydroxylamine hydrochloride was replaced with the same molar amount of diethyl amine hydrochloride. Absorption of the oxygen gas came near to termination after 1.5 hours of the reaction when about 2 m moles of oxygen had been absorbed. After keeping for additional 1.5 hours to complete the reaction, the reaction mixture was analyzed to find that the conversion of the TMP was 100% and the yield of the TMQ was 80.5% corresponding to a selectivity of 80.5%.

### Example 33.

The reaction conditions were substantially the same as in Example 32 except that the diethyl amine hydrochloride was replaced with the same molar amount of trimethyl amine hydrochloride. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 82.6% corresponding to a selectivity of 82.6%.

### Example 34.

The reaction conditions were substantially the same as in Example 32 except that the diethyl amine hydrochloride was replaced with the same molar amount of triethyl amine hydrochloride. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 83.1% corresponding to a selectivity of 83.1%.

### Example 35.

The reaction conditions were substantially the same as in Example 32 except that the diethyl amine hydrochloride was replaced with the same molar amount of N,N-diethyl glycine hydrochloride. Absorption of the oxygen gas came near to termination after 3 hours of the reaction when about 2 m moles of oxygen had been absorbed. After keeping for additional 2 hours to complete the reaction, the reaction mixture was analyzed to find that the conversion of the TMP was 100% and the yield of the TMQ was 79.1% corresponding to a selectivity of 79.1%.

### Example 36.

The reaction conditions were substantially the same as in Example 32 except that the diethyl amine hydrochloride was replaced with the same molar amount of propanolamine hydrochloride. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 86.9% corresponding to a selectivity of 86.9%.

### Example 37.

The reaction conditions were substantially the same as in Example 32 except that the diethyl amine hydrochloride was replaced with the same molar amount of diethanolamine hydrochloride. Absorption of the oxygen gas came near to termination after 3 hours of the reaction when about 2 m moles of oxygen had been absorbed. After keeping for additional 2 hours to complete the reaction, the reaction mixture was analyzed to find that the conversion of the TMP was 100% and the yield of the TMQ was 80.1% corresponding to a selectivity of 80.1%.

### Example 38.

The reaction conditions were substantially the same as in Example 32 except that the diethyl amine hydrochloride was replaced with the same molar amount of ethyl amine hydrobromide. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 78.1% corresponding to a selectivity of 78.1%.

### Example 39.

The reaction conditions were substantially the same as in Example 32 except that the diethyl amine hydrochloride was replaced with the same molar amount of diisopropyl amine hydrochloride. Absorption of the oxygen gas came near to termination after 4 hours of the reaction when about 2 m moles of oxygen had been absorbed. After keeping for additional 1 hour to complete the reaction, the reaction mixture was analyzed to find that the conversion of the TMP was 100% and the yield of the TMQ was 84.1% corresponding to a selectivity of 84.1%.

### Example 40.

The reaction conditions were substantially the same as in Example 32 except that the diethyl amine hydrochloride was replaced with the same molar amount of aniline hydrochloride. About 2 m moles of oxygen were absorbed during the reaction for 5 hours. The reaction mixture was analyzed to find that the conversion of the TMP was 100% and the yield of the TMQ was 69.6% corresponding to a selectivity of 69.6%.

### Example 41.

The reaction conditions were substantially the same as in Example 32 except that the n-hexyl alcohol was replaced with the same molar amount of n-octyl alcohol. Absorption of the oxygen gas came near to termination after 1.5 hours of the reaction when about 2 m moles of oxygen had been absorbed. After keeping for additional 1.5 hours to complete the reaction, the reaction mixture was analyzed to find that the conversion of the TMP was 100% and the yield of the TMQ was 76.2% corresponding to a selectivity of 76.2%.

### Example 42.

The reaction conditions were substantially the same as in Example 32 except that the n-hexyl alcohol was replaced with the same molar amount of cyclohexyl alcohol. About 2 m moles of oxygen were absorbed during the reaction for 5 hours. The reaction mixture was analyzed to find that the conversion of the TMP was 98.1% and the yield of the TMQ was 79.3% corresponding to a selectivity of 80.8%.

### Example 43.

The reaction conditions were substantially the same as in Example 32 except that the n-hexyl alcohol was replaced with the same molar amount of diacetone alcohol. About 2 m moles of oxygen were absorbed during the reaction for 5 hours. The reaction mixture was analyzed to find that the conversion of the TMP was 93.5% and the yield of the TMQ was 59.5% corresponding to a selectivity of 63.6%.

### Example 44.

The reaction conditions were substantially the same as in Example 32 except that the n-hexyl alcohol was replaced with the same molar amount of 2-octanol. Absorption of the oxygen gas came near to termination after 2 hours of the reaction when about 2 m moles of oxygen had been absorbed. After keeping for additional 3 hours to complete the reaction, the reaction mixture was analyzed to find that the conversion of the TMP was 100% and the yield of the TMQ was 77.3% corresponding to a selectivity of 77.3%.

### Example 45.

The reaction conditions were substantially the same as in Example 32 except that the diethyl amine hydrochloride was replaced with a combination of 0.2 m mole of triethyl amine and 0.017 ml of a 36% hydrochloric acid corresponding to 0.2 m mole of hydrogen chloride. Absorption of the oxygen gas came near to termination after 2.5 hours of the reaction when about 2 m moles of oxygen had been absorbed. After keeping for additional 2.5 hours to complete the reaction, the reaction mixture was analyzed to find that the conversion of the TMP was 100% and the yield of the TMQ was 88.6% corresponding to a selectivity of 88.6%.

### Example 46.

The reaction conditions were substantially the same as in Example 32 except that the reaction temperature was 40 °C instead of 60 °C. About 2 m moles of oxygen were absorbed during the reaction for 6 hours. The reaction mixture was analyzed to find that the conversion of the TMP was 100% and the yield of the TMQ was 83.1% corresponding to a selectivity of 83.1%.

### Example 47.

The reaction conditions were substantially the same as in Example 32 except that the amounts of TMP, copper (II) chloride dihydrate and diethylamine hydrochloride were 3 m moles, 0.1 m mole and 0.1 m mole, respectively. Absorption of the oxygen gas came near to termination after 6 hours of the reaction when about 3 m moles of oxygen had been absorbed. After keeping for additional 3 hours to complete the reaction, the reaction mixture was analyzed to find that the conversion of the TMP was 100% and the yield of the TMQ was 89.4% corresponding to a selectivity of 89.4%.

### Example 48.

The reaction conditions were substantially the same as in Example 32 except that the copper (II) chloride dihydrate and diethyl amine hydrochloride was replaced each with the same molar amount of anhydrous copper (II) chloride and triethyl amine hydrochloride, respectivley. Absorption of the oxygen gas came near to termination after 2.5 hours of the reaction when about 2 m moles of oxygen had been absorbed. After keeping for additional 2.5 hours to complete the reaction, the reaction mixture was analyzed to find that the conversion of the TMP was 100% and the yield of the TMQ was 86.9% corresponding to a selectivity of 86.9%.

### Example 49.

The reaction conditions were substantially the same as in Example 32 except that the diethyl amine hydrochloride was replaced with a combination of 0.2 m mole of morpholine and 0.017 ml of a 36% hydrochloric acid corresponding to 0.2 m mole of hydrogen chloride. Absorption of the oxygen gas came near to termination after 2 hours of the reaction when about 2 m moles of oxygen had been absorbed. After keeping for additional 2 hours to complete the reaction, the reaction mixture was analyzed to find that the conversion of the TMP was 100% and the yield of the TMQ was 72.1% corresponding to a selectivity of 72.1%.

### Comparative Example 5.

The reaction conditions were substantially the same as in Example 36 except that the propanolamine hydrochloride was replaced with the same molar amount of free propanolamine. After 4 hours of the reaction, the reaction mixture was analyzed to find that the conversion of the TMP was 100% and the yield of the TMQ was 30.0% corresponding to a selectivity of 30.0%.

### Comparative Example 6.

The reaction conditions were substantially the same as in Example 37 except that the diethanolamine hydrochloride was replaced with the same molar amount of free diethanolamine. After 4 hours of the reaction, the reaction mixture was analyzed to find that the conversion of the TMP was 100% and the yield of the TMQ was 30.2% corresponding to a selectivity of 30.2%.

### Comparative Example 7.

The reaction conditions were substantially the same as in Example 48 except that the triethylamine hydrochloride was replaced with the same molar amount of tetraethylammonium chloride. About 1.4 m moles of oxygen were absorbed during the reaction for 5 hours. The reaction mixture was analyzed to find that the conversion of the TMP was 82.5% and the yield of the TMQ was 57.7% corresponding to a selectivity of 69.2%.

### Comparative Example 8.

The reaction conditions were substantially the same as in Example 45 excepting omission of the hydrochloric acid. About 2 m moles of oxygen were absorbed during the reaction for 5 hours. The reaction mixture was analyzed to find that the conversion of the TMP was 100% and the yield of the TMQ was 22.3% corresponding to a selectivity of 22.3%.

### Example 50.

Several experiments were conducted each in substantially the same experimental conditions as in Example 1 except that the amounts of the copper (II) chloride dihydrate and hydroxylamine hydrochloride were each 0.2 m mole instead of 0.1 m mole and the n-hexyl alcohol was replaced with the same volume of a different alcohol. The overall reaction time was 3 hours or 4 hours and conversion of the TMP was 100% in each of the experiments. When the alcohol was isopropyl alcohol, sec-butyl alcohol, tert-butyl alcohol, pentan-2-ol, pentan-3-ol or tert-amyl alcohol, the yield of the product TMQ was 90.6%, 88.8%, 91.1%, 89.3%, 92.4% or 92.6%, respectively. When the branched alcohol used above was replaced with the same volume of ethyl alcohol, n-propyl alcohol or n-hexyl alcohol, the yield of the product TMQ was 84.5%, 86.0% or 86.0%, respectively.

### Example 51.

Several experiments were conducted each in substantially the same manner as in Example 50 excepting replacement of 0.2 m mole of hydroxylamine hydrochloride with 0.1 m mole of hydroxylamine sulfate. When the alcohol was isopropyl alcohol, sec-butyl alcohol, tert-butyl alcohol, pentan-2-ol, pentan-3-ol or tert-amyl alcohol, the yield of the product TMQ was 89.8%, 92.3%, 90.6%, 90.6%, 91.8% or 92.7%, respectively. When the branched alcohol used above was replaced with the same volume of ethyl alcohol, n-propyl alcohol or n-hexyl alcohol, the yield of the product TMQ was 83.6%, 86.6% or 88.8%, respectively.

### Example 52.

Several experiments were conducted each in substantially the same manner as in Example 50 excepting replacement of 0.2 m mole of hydroxylamine hydrochloride with a combination of 0.2 m mole of acetone oxime and 0.2 m mole of hydrogen chloride added in the form of a 36% hydrochloric acid. The overall reaction time was 4 hours in each experiment. When the alcohol was isopropyl alcohol, sec-butyl alcohol, tert-butyl alcohol, pentan-2-ol, pentan-3-ol or tert-amyl alcohol, the yield of the product TMQ was 91.0%, 93.7%, 95.0%, 91.4%, 92.1% or 94.5%, respectively. When the branched alcohol used above was replaced with the same volume of ethyl alcohol, n-propyl alcohol or n-hexyl alcohol, the yield of the product TMQ was 76.5%, 82.6% or 84.5%, respectively.

### Example 53.

Several experiments were conducted each in substantially the same manner as in Example 50 excepting replacement of 0.2 m mole of hydroxylamine hydrochloride with 0.2 m mole of diethylamine hydrochloride. The overall reaction time was 5 hours in each experiment. When the alcohol was sec-butyl alcohol, pentan-2-ol, pentan-3-ol or tert-amyl alcohol, the yield of the product TMQ was 74.1%, 86.5%, 80.0% or 84.0%, respectively. When the branched alcohol used above was replaced with the same volume of ethyl alcohol or n-propyl alcohol, the yield of the product TMQ was 60.2% or 71.3%, respectively.

### Example 54.

Several experiments were conducted each in substantially the same manner as in Example 50 excepting replacement of 2 ml of the alcohol with a combination of 1 ml of toluene and 1 ml of an alcohol. When the alcohol was ethyl alcohol, n-propyl alcohol, isopropyl alcohol, n-butyl alcohol, sec-butyl alcohol, tert-butyl alcohol, n-amyl alcohol or tert-amyl alcohol, the yield of the product TMQ was 86.7%, 88.4%, 91.9%, 87.0%, 91.5%, 91.2%, 88.2% or 91.9%, respectively.

Further, the same experimental procedure as above was repeated excepting replacement of the toluene with the same volume of benzene. When the alcohol was ethyl alcohol or isopropyl alcohol, the yield of the product TMQ was 86.7% or 89.9%, respectively.

### Example 55.

Several experiments were conducted each in substantially the same manner as in Example 50 excepting replacement of 0.2 m mole of hydroxylamine hydrochloride with 0.1 m mole of hydroxylamine sulfate and replacement of 2 ml of the alcohol with a combination of 1 ml of toluene and 1 ml of an alcohol. When the alcohol was ethyl alcohol, n-propyl alcohol, isopropyl alcohol, n-butyl alcohol, sec-butyl alcohol, tert-butyl alcohol, n-amyl alcohol or tert-amyl alcohol, the yield of the product TMQ was 88.8%, 90.7%, 93.0%, 89.2%, 93.4%, 89.6%, 89.0% or 91.9%, respectively.

Further, the same experimental procedure as above was repeated excepting replacement of the toluene with the same volume of benzene. When the alcohol was ethyl alcohol or isopropyl alcohol, the yield of the product TMQ was 88.2% or 91.3%, respectively.

### Example 56.

Several experiments were conducted each in substantially the same manner as in Example 50 excepting replacement of 0.2 m mole of hydroxylamine hydrochloride with a combination of 0.2 m mole of acetone oxime and 0.2 m mole of hydrogen chloride added in the form of a 36% hydrochloric acid and replacement of 2 ml of the alcohol with a combination of 1 ml of toluene and 1 ml of an alcohol. When the alcohol was ethyl alcohol, n-propyl alcohol, isopropyl alcohol, n-butyl alcohol, sec-butyl alcohol, tert-butyl alcohol, n-amyl alcohol or tert-amyl alcohol, the yield of the product TMQ was 88.6%, 91.1%, 92.6%, 90.0%, 92.9%, 91.2%, 90.0% or 92.6%, respectively.

Further, the same experimental procedure as above was repeated excepting replacement of the toluene with the same volume of benzene. When the alcohol was ethyl alcohol or isopropyl alcohol, the yield of the product TMQ was 85.2% or 93.6%, respectively.

### Example 57.

Several experiments were conducted each in substantially the same manner as in Example 50 excepting replacement of 0.2 m mole of hydroxylamine hydrochloride with 0.2 m mole of diethylamine hydrochloride and replacement of 2 ml of the alcohol with a combination of 1 ml of toluene and 1 ml of an alcohol. When the alcohol was ethyl alcohol, n-propyl alcohol, isopropyl alcohol, n-butyl alcohol, sec-butyl alcohol, tert-butyl alcohol, n-amyl alcohol or tert-amyl alcohol, the yield of the product TMQ was 77.5%, 86.4%, 89.4%, 86.4%, 92.0%, 85.8%, 87.1% or 85.8%, respectively.

Further, the same experimental procedure as above was repeated excepting replacement of the toluene with the same volume of benzene. When the alcohol was ethyl alcohol or isopropyl alcohol, the yield of the product TMQ was 78.5% or 87.6%, respectively.

### Example 58.

Three experiments were conducted each under substantially the same conditions as in Example 54 except that the solvent was a mixture of toluene and isopropyl alcohol in different proportions of (1.5 ml + 0.5 ml), (1.3 ml + 0.7 ml) and (0.5 ml + 1.5 ml) to give an yield of the product TMQ of 93.1%, 91/0% and 88.7%, respectively. Note that the yield was 91.9% when the solvent was a mixture of 1.0 ml toluene and 1.0 ml isopropyl alcohol. The conversion of the starting TMP was 100% in each experiment after 3 hours of the overall reaction time.

### Comparative Example 9.

The experimental conditions were substantially the same as in Example 54 except that the solvent was a mixture of 1.0 ml of toluene and 1.0 ml of isopropyl alcohol and 0.2 m mole of the hydroxylamine hydrochloride was replaced with 0.2 m mole of lithium chloride. After 5 hours of the reaction, the conversion of the starting TMP was 68.9% and the yield of the product TMQ was 38.9%.

### Example 59.

Three experiments were conducted each in substantially the same manner as in Example 50 except that the solvent was a mixture of 1.0 ml of toluene and 1.0 ml of isopropyl alcohol and the catalyst system was a combination of 0.1 m mole of copper (II) chloride dihydrate and 0.2 m mole of hydroxylamine hydrochloride, 0.1 m mole of hydroxylamine sulfate or a 0.2 m mole of acetone oxime as combined with 0.2 m mole of hydrogen chloride in the form of a 36% hydrochloric acid to give a yield of the product TMQ of 92.7%, 93.2% or 93.3%, respectively. The conversion of the starting TMP was 100% in each of the experiments after 3-4 hours of the overall reaction time.

### Example 60.

The experimental conditions were substantially the same as in the third experiment in Example 59 excepting replacement of the copper (II) chloride dihydrate with the same molar amount of copper acetate monohydrate. The reaction conducted for 5 hours of the overall reaction time gave 100% conversion of the starting TMP and 83.3% yield of the product TMQ.

### Example 61.

A glass-made reaction vessel of 50 ml capacity was charged with 5.0 m moles of TMP, an aqueous solution of 1.0 m mole of copper (II) chloride dihydrate and 2.5 m moles of hydroxylamine hydrochloride dissolved in 5 ml of water, 5 ml of tert-butyl alcohol and 5 ml of toluene to form a reaction mixture which was agitated vigorously at a temperature of 60 °C under a constant oxygen pressure of 860 mmHg for 1 hour to effect the reaction with periodical measurement of the volume of absorbed oxygen gas using a gas burette.

After completion of the reaction, the reaction mixture was separated into an aqueous solution and an organic solution. The aqueous solution was subjected to extraction with methylene chloride and the extract solution was combined with the organic solution from the reaction mixture. The combined organic solution was washed with water and dried followed by a gas chromatographic analysis for the content of TMQ as the product.

The aqueous solution recovered from the reaction mixture was admixed with 2.5 m moles of hydroxylamine hydrochloride, 5 ml of tert-butyl alcohol, 5 ml of toluene and 5.0 m moles of TMP to form a reaction mixture for a second run which was conducted for 1 hour under the same conditions as in the above described first run. In this manner, the aqueous solution containing the copper salt recovered from a run of the oxidation reaction was recycled to the next run each time by adding fresh portions of hydroxylamine hydrochloride, tert-butyl alcohol, toluene and TMP. Table 1 below shows the yield of the product TMQ in each of the first nine runs. The conversion of the starting TMP was 100% in each run.

The aqueous solution recovered from the ninth run of the reaction was recycled to the tenth run with addition of 5 ml of tert-butyl alcohol and 5.0 m moles of TMP alone but without addition of a fresh portion of hydroxylamine hydrochloride. The result of this tenth run was that absolutely no TMQ was found in the reaction mixture after the reaction with only 4.0% conversion of the starting TMP.

**Table 1**

| Run No. | Yield of TMQ, % |
|---|---|
| 1 | 63.2 |
| 2 | 67.5 |
| 3 | 86.2 |
| 4 | 86.6 |
| 5 | 88.7 |
| 6 | 89.7 |
| 7 | 90.5 |
| 8 | 91.0 |
| 9 | 92.4 |

### Comparative Example 10.

The same experimental procedure as in the first run of Example 61 was repeated excepting replacement of the hydroxylamine hydrochloride with the same molar amount of lithium chloride. The result was that the yield of TMQ was only 1.2% with 81.9% of the conversion of the starting TMP.

### Example 62.

Two experiments were conducted each by introducing, into a glass-made reaction vessel of 50 ml capacity, 10 m moles of TMP, 1.0 m mole of copper (II) chloride dihydrate, 5.0 m moles of hydroxylamine hydrochloride or 5.0 m moles of acetone oxime as combined with 5.0 m moles of hydrogen chloride in the form of a 36% hydrochloric acid dissolved in 10 ml of water, and 10 ml of n-butyl alcohol to form a reaction mixture which was agitated vigorously at a temperature of 60 °C under a constant oxygen pressure of 860 mmHg for 2 hours to effect the reaction with periodical measurement of the volume of absorbed oxygen gas using a gas burette.

After completion of the reaction, the reaction mixture was separated into an aqueous solution and an organic solution. The aqueous solution was subjected to extraction with methylene chloride and the extract solution was combined with the organic solution from the reaction mixture. The combined organic solution was washed with water and dried followed by a gas chromatographic analysis for the content of TMQ as the product.

The aqueous solution recovered from the reaction mixture was admixed with 5.0 m moles of hydroxylamine hydrochloride or 5.0 m moles of acetone oxime as combined with 5.0 m moles of hydrogen chloride in the form of a 36% hydrochloric acid, 10 ml of n-butyl alcohol and 10 m moles of TMP to form a reaction mixture for a second run which was conducted for 2 hours under the same conditions as in the above described first run. In this manner, the aqueous solution containing the copper salt recovered from a run of the oxidation reaction was recycled to the next run each time by adding fresh portions of hydroxylamine hydrochloride, n-butyl alcohol and TMP. Table 2 below shows the yield of the product TMQ in each of the runs conducted to the fourth run in the series of experiments using hydroxylamine hydrochloride and a combination of acetone oxime and hydrochloric acid, respectively. The conversion of the starting TMP was 100% in each run.

**Table 2**

| Run No. | Yield of TMQ, % | |
|---|---|---|
| | Hydroxylamine hydrochloride | Acetone oxime + hydrogen chloride |
| 1 | 73.7 | 75.8 |
| 2 | 80.8 | 78.3 |
| 3 | 78.0 | 80.7 |
| 4 | 74.0 | 76.7 |

### Comparative Example 11.

Three experiments were conducted each under the same experimental conditions as in Example 54 excepting replacement of the combination of an alcoholic solvent and an aromatic hydrocarbon solvent with 2 ml of benzene alone, 2 ml of toluene alone and a combination of 1 ml of toluene and 1 ml of acetone. The conversion of the starting TMP was 5% in each of these experiments but absolutely no TMQ could be found in the reaction mixture after the end of the reaction time.

## Claims

1. A method for the preparation of 2,3,5-trimethyl benzoquinone by the oxidation of 2,3,6-trimethyl phenol which comprises the step of:
contacting a reaction mixture containing 2,3,6-trimethyl phenol dissolved in an alcoholic organic solvent with molecular oxygen in the presence of a catalyst system which is a combination of a copper compound and a nitrogen-containing compound selected from
(a) salts of a hydroxylamine compound with an inorganic acid,
(b-1) oxime compounds,
(b-2) combinations of an oxime compound and an inorganic acid, and
(c) salts of an amine compound with an inorganic acid.

2. A method as claimed in Claim 1 wherein the alcoholic organic solvent is an aliphatic alcohol having 1 to 10 carbon atoms in a molecule.

3. A method as claimed in Claim 2 wherein the aliphatic alcohol is a primary alcohol having 4 to 10 carbon atoms in a molecule.

4. A method as claimed in Claim 2 wherein the aliphatic alcohol has 3 to 6 carbon atoms in a molecule and a branched molecular structure.

5. A method as claimed in Claim 4 wherein the aliphatic alcohol is a tertiary alcohol.

6. A method as claimed in Claim 1 wherein the alcoholic organic solvent is a mixture of an aliphatic alcohol having 1 to 6 carbon atoms in a molecule and an aromatic hydrocarbon solvent.

7. A method as claimed in Claim 6 wherein the aliphatic alcohol and the aromatic hydrocarbon solvent are mixed in a proportion in the range from 0.2:1 to 1.5:1 by volume.

8. A method as claimed in Claim 6 or Claim 7 wherein the aromatic hydrocarbon solvent is toluene or benzene.

9. A method as claimed in any one of the preceding claims wherein the copper compound is a chloride of copper.

10. A method as claimed in any one of the preceding claims wherein the hydroxylamine compound is selected from hydroxylamine, hydroxy urea and N,N-dialkyl hydroxylamines.

11. A method as claimed in any one of the preceding claims wherein the oxime compound is selected from acetaldoxime, benzaldoxime, acetone oxime and 2-butanone oxime.

12. A method as claimed in any one of the preceding claims wherein the amount of the copper compound is in the range from 0.01 to 0.5 mole per mole of the 2,3,6-trimethyl phenol.

13. A method as claimed in any one of the preceding claims wherein the amount of the nitrogen-containing compound is in the range from 0.2 to 5 moles per mole of the copper compound.

14. A method as claimed in Claim 1 wherein the oxidation reaction is performed at a temperature in the range from 40°C to 150°C.

15. A method as claimed in any one of the preceding claims wherein the reaction mixture contains water in such an amount that the reaction mixture is heterogeneous consisting of an aqueous phase and an organic phase.

16. A method as claimed in any one of the preceding claims wherein the inorganic acid to form a salt with the hydroxylamine compound, oxime compound or amine compound is hydrochloric acid or sulfuric acid.

## Patentansprüche

1. Verfahren zur Herstellung von 2,3,5-Trimethylbenzchinon durch Oxydation von 2,3,6-Trimethylphenol in folgenden Schritten:
Zusammenbringen eines Reaktionsgemischs, das in einem alkoholischen Lösungsmittel gelöstes 2,3,6-Trimethylphenol enthält, mit molekularem Sauerstoff in Gegenwart eines Katalysatorsystems in Form einer Kombination aus einer Kupferverbindung und einer Stickstoff enthaltenden Verbindung, die ausgewählt ist aus
(a) Salzen einer Hydroxylaminverbindung mit einer anorganischen Säure,
(b-1) Oximverbindungen,
(b-2) Kombinationen einer Oximverbindung mit einer anorganischen Säure und
(c) Salzen einer Aminverbindung mit einer anorganischen Säure.

2. Verfahren nach Anspruch 1, wobei das alkoholische organische Lösungsmittel ein aliphatischer Alkohol mit 1 bis 10 Kohlenstoffatomen im Molekül ist.

3. Verfahren nach Anspruch 2, wobei der aliphatische Alkohol ein primärer Alkohol mit 4 bis 10 Kohlenstoffatomen im Molekül ist.

4. Verfahren nach Anspruch 2, wobei der aliphatische Alkohol 3 bis 6 Kohlenstoffatome im Molekül und eine verzweigte Molekularstruktur hat.

5. Verfahren nach Anspruch 4, wobei der aliphatische Alkohol ein tertiärer Alkohol ist.

6. Verfahren nach Anspruch 1, wobei das alkoholische organische Lösungsmittel ein Gemisch aus einem aliphatischen Alkohol mit 1 bis 6 Kohlenstoffatomen im Molekül und einem aromatischen Kohlenwasserstofflösungsmittel ist.

7. Verfahren nach Anspruch 6, wobei der aliphatische Alkohol und das aromatische Kohlenwasserstofflösungsmittel in einem Verhältnis im Bereich von 0.2:1 bis 1.5:1 auf Volumenbasis gemischt werden.

8. Verfahren nach Anspruch 6 oder 7, wobei das aromatische Kohlenwasserstofflösungsmittel Toluol oder Benzol ist.

9. Verfahren nach einem der vorhergehenden Ansprüche, wobei die Kupferverbindung ein Kupferchlorid ist.

10. Verfahren nach einem der vorhergehenden Ansprüche, wobei die Hydroxylaminverbindung ausgewählt ist aus Hydroxylamin, Hydroxyharnstoff und N,N-dialkylhydroxylaminen.

11. Verfahren nach einem der vorhergehenden Ansprüche, wobei die Oximverbindung ausgewählt ist aus Acetaldoxim, Benzalkoxim, Azetonoxim und 2-butanonoxim.

12. Verfahren nach einem der vorhergehenden Ansprüche, wobei die Menge an Kupferverbindung im Bereich von 0.01 bis 0.5 Mol je Mol an 2,3,6-Trimethylphenol liegt.

13. Verfahren nach einem der vorhergehenden Ansprüche, wobei die Menge an stickstoffhaltiger Kupferverbindung im Bereich von 0.2 bis 5 Mol je Mol der Kupferverbindung liegt.

14. Verfahren nach Anspruch 1, wobei die Oxidationsreaktion in einem Temperaturbereich von 40° bis 150°C durchgeführt wird.

15. Verfahren nach einem der vorhergehenden Ansprüche, wobei das Reaktionsgemisch Wasser in solcher Menge enthält, daß das Reaktionsgemisch heterogen ist und aus einer wäßrigen Phase und einer organischen Phase besteht.

16. Verfahren nach einem der vorhergehenden Ansprüche, wobei die anorganische Säure zur Bildung eines Salzes mit der Hydroxylaminverbindung, der Oximverbindung oder der Aminverbindung Salzsäure oder Schwefelsäure ist.

## Revendications

1. Méthode pour la préparation de triméthyl 2,3,5-benzoquinone par l'oxydation de triméthyl 2,3,6-phénol, qui comprend les étapes de :
mettre un mélange réactionnel contenant le triméthyl 2,3,6-phénol dissous dans un solvant organique alcoolisé en contact avec de l'oxygène moléculaire en présence d'un système catalyseur qui est une combinaison d'un composé de cuivre et d'un composé contenant de l'azote choisi parmi
(a) des sels d'un composé d'hydroxylamine avec un acide inorganique,
(b-1) des composés d'oxime,
(b-2) des combinaisons d'un composé d'oxime et d'un acide inorganique et
(c) des sels d'un composé d'amine avec un acide inorganique.

2. Méthode selon la revendication 1, où le solvant organique alcoolisé est un alcool aliphatique ayant 1 à 10 atomes de carbone dans une molécule.

3. Méthode selon la revendication 2, où l'alcool aliphatique est un alcool primaire ayant 4 à 10 atomes de carbone dans une molécule.

4. Méthode selon la revendication 2, où l'alcool aliphatique a 3 à 6 atomes de carbone dans une molécule et une structure moléculaire ramifiée.

5. Méthode selon la revendication 4, où l'alcool aliphatique est un alcool tertiaire.

6. Méthode selon la revendication 1, où le solvant organique alcoolisé est un mélange d'un alcool aliphatique ayant 1 à 6 atomes de carbone dans une molécule et d'un hydrocarbure solvant aromatique.

7. Méthode selon la revendication 6, où l'alcool aliphatique et l'hydrocarbure solvant aromatique sont mélangés à une proportion comprise entre 0,2:1 et 1,5:1 en volume.

8. Méthode selon la revendication 6 ou la revendication 7, où l'hydrocarbure solvant aromatique est le toluène ou le benzène.

9. Méthode selon l'une quelconque des revendications précédentes, où le composé de cuivre est le chlorure de cuivre.

10. Méthode selon l'une quelconque des revendications précédentes, où le composé d'hydroxylamine est choisi parmi l'hydroxylamine, l'hydroxy urée et des N,N-dialkyl hydroxylamines.

11. méthode selon l'une quelconque des revendications précédentes, où le composé d'oxime est choisi parmi acétaldoxime, benzaldoxime, acétone oxime et 2-butanone oxime.

12. Méthode selon l'une quelconque des revendications précédentes, où la quantité du composé de cuivre est comprise entre 0,01 et 0,5 mole par mole de triméthyl 2,3,6-phénol.

13. Méthode selon l'une quelconque des revendications précédentes, où la quantité du composé contenant de l'azote est comprise entre 0,2 et 5 moles par mole du composé de cuivre.

14. Méthode selon la revendication 1, où la réaction d'oxydation est accomplie à une température comprise entre 40°C et 150°C.

15. Méthode selon l'une quelconque des revendications précédentes, où le mélange réactionnel contient de l'eau en une quantité telle que le mélange réactionnel soit hétérogène, consistant en une phase aqueuse et une phase organique.

16. Méthode selon l'une quelconque des revendications précédentes, où l'acide inorganique pour former un sel avec le composé d'hydroxylamine, le composé d'oxime ou le composé d'amine est l'acide chlorhydrique ou l'acide sulfurique.
